# EUROPEAN PATENT SPECIFICATION

(11) **EP 1 752 156 B1**
(45) Date of publication and mention of the grant of the patent: **30.07.2014**
(21) Application number: 05737116.3
(22) Date of filing: 26.04.2005
(51) Int. Cl.: A23L 1/30, A61K 36/42, A61Q 7/00, A61Q 19/00, A61Q 5/02, A61Q 5/12

(54) **ACTIVE OXYGEN ELIMINATOR AND MOISTURIZER CONTAINING WILD WATERMELON EXTRACT**
AKTIVER SAUERSTOFF-ELIMINATOR UND FEUCHTIGKEITSSPENDER MIT EINEM EXTRAKT AUS WILDEN WASSERMELONEN
ÉLIMINATEUR D'OXYGÈNE ACTIF ET HYDRATNANT CONTENANT DE L'EXTRAIT DE MELON SAUVAGE

(30) Priority: 30.04.2004 JP 2004135241
(43) Date of publication of application: 14.02.2007
(73) Proprietor: National University Corporation Nara Institute of Science and Technology, Ikoma-shi, Nara 630-0192 (JP)
(72) Inventor: AKASHI, Kinya, Nara-shi Nara 6310041 (JP); YOKOTA, Akiho, 6300246 Nara (JP)
(74) Representative: Casalonga, Axel
(86) International application number: PCT/JP2005/007909
(87) International publication number: WO 2005/105126

(56) References cited:
- CN-A- 1 422 650
- JP-A- 10 025 235
- JP-A- 2000 212 027
- JP-A- 2001 226 249
- JP-A- 2001 226 249
- JP-A- 2002 226 370
- US-A- 2 298 328
- US-A- 5 932 230
- SEOK JOONG KIM ET AL: "Screening for superoxide dismutase-like compounds and its activators in extracts of fruits and vegetables." BIOSCIENCE, BIOTECHNOLOGY, AND BIOCHEMISTRY 1994 CORRESPONDENCE (REPRINT) ADDRESS, DAESEOK HAN, AGRIC. PRODUCTS UTIL. DIV., KOREA FOOD RES. INST., BUNDANG, SONGNAM, KYONGGI, KOREA, vol. 58, no. 12, 1994, page 2263, XP002540334
- BURATTI S ET AL: "Rapid electrochemical method for the evaluation of the antioxidant power of some lipophilic food extracts." JOURNAL OF AGRICULTURAL AND FOOD CHEMISTRY 49 (11) 5136-5141 2001 CORRESPONDENCE (REPRINT) ADDRESS, O. V. BRENNA, DEP. OF FOOD SCI., UNIV. OF MILAN, 20133 MILAN, ITALY. TEL. +39 02 5835 6632. FAX +39 02 5835 6623. E-MAIL ORESTE.BRENNA(A)UNIMI.IT, 2001, XP002540335
- EDWARDS ALISON J ET AL: "Consumption of watermelon juice increases plasma concentrations of lycopene and beta-carotene in humans." THE JOURNAL OF NUTRITION APR 2003, vol. 133, no. 4, April 2003 (2003-04), pages 1043-1050, XP002540336 ISSN: 0022-3166
- AKASHI K ET AL: "Citrulline functions as the efficient hydroxyl radical scavenger: Implication for the drought-tolerance of wild watermelon plant" PHOTOSYNTHESIS RESEARCH, vol. 69, no. 1-3, 2001, page 170, XP002540337 & 12TH INTERNATIONAL CONGRESS ON PHOTOSYNTHESIS; BRISBANE, AUSTRALIA; AUGUST 18-23, 2001 ISSN: 0166-8595
- AKASHI K. ET AL: 'Citrulline, a novel compatible solute in drought-tolerant wild watermelon leaves, is an efficient hydroxyl radical scavenger.' FEBS LETTERS. vol. 508, 2001, pages 438 - 442, XP004324285
- MIYAKE O. ET AL: 'Yaseishu to Saibaishu Suika deno Kokaguteki II Ryoshi Shirutsu to Kassei Sanso Shokyo Koso Kassei no Kanso Stress Oto.' THE JAPANESE SOCIETY OF PLANT PHYSIOLOGIST. vol. 40, 2000, page 189
- NISHIMURA N. ET AL: 'Yaseishu Suika de Kanso Yudo Sareru Metallothionein no Kino Kaiseki.' vol. 41, 2001, page 203, XP008142296
- NANASATO Y. ET AL: 'Yaseishu Suika ni Okeru Kyoko Kanso Stress Yudosei 2 Hem-gataen to Chrome b561 no Kino Kaiseki.' vol. 45, March 2004, page 167, XP008142295
- BOURJOIS: 'Hydra-été Shower Gel', [Online] 31 March 2003, GNPD, XP055049621 Retrieved from the Internet: <URL:http://www.gnpd.com/sinatra/gnpd/searc h_results&search_id=STzP4MFY5O/&item_id=194 806> [retrieved on 2013-01-15]
- JANINE E. VICTOR (WITH ADDITIONS FROM J. M. SUTTIE): 'Citrullus lanatus (Thunb.) Matsum. & Nakai', [Online] 14 June 2004, pages 1 - 2, XP055049921 Retrieved from the Internet: <URL:http://www.fao.org/ag/AGP/AGPC/doc/Gba se/Safricadata/citlan.htm> [retrieved on 2013-01-16]
- SMIRNOFF N ET AL: "Hydroxyl radical scavenging activity of compatible solutes", PHYTOCHEMISTRY, PERGAMON PRESS, GB, vol. 28, no. 4, 1 January 1989 (1989-01-01), pages 1057-1060, XP026616533, ISSN: 0031-9422, DOI: 10.1016/0031-9422(89)80182-7 [retrieved on 1989-01-01]
- DATABASE BIOSIS [Online] BIOSCIENCES INFORMATION SERVICE, PHILADELPHIA, PA, US July 2000 KAWASAKI SHINJI ET AL: 'Responses of wild watermelon to drought stress: Accumulation of an ArgE homologue and citrulline in leaves during water deficits' Database accession no. PREV200000377121 & PLANT AND CELL PHYSIOLOGY, vol. 41, no. 7, July 2000 (2000-07), pages 864-873, ISSN: 0032-0781

## Description

### Technical Field

The present invention relates to the moisturizing agent to moisturize, cosmetics, hair care products, bathwater agents and hygiene products containing these.

### Background Art

The active oxygen such as hydroxyl radical, etc., is a main cause in triggering various diseases and aging, moreover causing skin problems such as freckles and age spots. The addition of antioxidant substances in cosmetics is wide spread and in such cases, mainly a chemically synthesized artificial compound is often used. But these groups of antioxidant substances often have had side-effects on the human body, or have problems of severe light aging and auto-oxidation. Therefore, it has been desired to develop a naturally-derived antioxidant with with a high degree of safety.

Conventionally, as naturally-derived antioxidants, citrulline is known, for example, and there exists an invention of an active oxygen scavenging agent and cosmetic compositions containing citrulline (Patent Literature 1). Citrulline has excellent active oxygen-scavenging ability and wild watermelon native to Botswana contains a significant quantity of citrulline. The active oxygen-scavenging ability of wild watermelons was considered to derive from citrulline. However, as its addition quantity could be less if there is a more active one than the active oxygen-scavenging agent containing citrulline, development of a composition having higher activity of active oxygen scavenging ability had been expected.

Moreover, as the extract of cultivated watermelons contains sucrose, it could not be directly used for cosmetics. So, the raw materials of watermelon to make juice having low sucrose content have been desired.

Moreover, it has been desired to develop cosmetics, bathwater agents and hair care products having a high degree of safety and a good shelf life and effectively protecting the human body from toxic light and toxic oxygen.

Patent Literature 1: Japanese Published Unexamined Patent Application No. 2002-226370

### Disclosure of the Invention

### Problems to be Solved by the Invention

The present invention provides use of compositions containing the extract of wild watermelon native to Botswana. More specifically, it provides use of the moisturizing agent containing the extract of wild watermelon native to Botswana.

### Means for Solving the Problems

The inventors have found that there is a strong active oxygen scavenger activity in wild watermelon of Botswana, as a result of devoting themselves to research to solve the problem.

This wild watermelon accumulates a significant amount of citrulline by drying stress, etc. As it is known that citrulline has active oxygen scavenging ability (Patent Literature 1), it has been thought that the active oxygen scavenging ability of wild watermelon derives from citrulline. However, the inventors measured the active oxygen scavenging activity of the juice of wild watermelon itself and showed active oxygen scavenging activity about ten times higher compared to the corresponding concentration of the citrulline aqueous solution. Consequently, it became clear that active oxygen scavenging activity of wild watermelon juice depends on other components more than citrulline. Namely, it was found that the activity of active oxygen scavenging is 10 times higher in the case which wild watermelon juice was used itself as the active oxygen scavenging agent than in the case in which a corresponding concentration of citrulline which is contained in wild watermelon juice was used. In addition, inventors have found that the wild watermelon extract has a rich moisturizing effect with the active oxygen scavenging ability and completed the invention.

The present invention is directed to the
(1) use, as moisturizing agent, of the *Citrullus lanatus* (wild watermelon) extract native to Botswana,
   - the wild watermelon extract being obtained by using *Citrullus lanatus* flesh, with the exception of hull, endothelium and seeds,
   wherein the total of each concentration of fructose, glucose and sucrose is 2% or less weight/weight percent and
   wherein the *Citrullus lanatus* (wild watermelon) extract has an oxygen scavenging activity about ten times higher than an aqueous solution containing only citrulline of the same concentration and
   wherein the *Citrullus lanatus* (wild watermelon) extract has a rich moisture comparable to glycerin.
(2) The use of claim 1, wherein the content rate of sugar in the flesh of *Citrullus lanatus* (wild watermelon) is 0.88% fructose, 0.45% glucose, and 0.05% or less of sucrose.
(3) The use as moisturizing agent, of the *Citrullus lanatus* (wild watermelon) extract of claim 1 or 2, for the preparation of a moisturizing cosmetic composition.
(4) The use according to any of claims 1 to 3, characterized in that the moisturizing agent is used to prepare a shampoo, a conditioner, a bathwater or a hair growth tonic.

### Effects of the Invention

According to the present disclosure the use of the active oxygen scavenging agent with high active oxygen scavenging activity, moisture rich moisturizing agent is provided.

### DESCRIPTION OF THE PREFERRED EMBODIMENTS

Hereinafter, examples of the present invention are described but the illustrative embodiment is not limited to these.

In the present invention 'wild watermelon' means wild watermelon (Citrullus lanatus) native to Bostwana which grows naturally in the Kalahari Desert, in Africa and the available watermelon in the present invention among the progeny watermelon varieties by crossbreeding of these, including one which grows artificially in a field, etc. 'The available watermelon in the present invention' means that the extract of watermelon flesh has more than twice or higher the scavenging activity of active oxygen than a single citrulline aqueous solution of the same concentration as the citrulline concentration of the extract of such watermelon, and that the total of each concentration of fructose, glucose and sucrose is 4% or less (weight/weight percent. Same as above). More preferably, it means that the extract of watermelon flesh has more than five times higher scavenging activity of the active oxygen than a single citrulline aqueous solution of the same concentration as the citrulline concentration of the extract of watermelon and that the total of each concentration of fructose, glucose and sucrose is 2% by weight or less. As Bushmen have eaten wild watermelons native to Botswana for a long time, its safety as a food is considered to not be an issue.

It is reported that the content rate of sugar in the flesh of the cultivated variety of watermelon grown in Japan, etc., is 5.03% fructose, 1.57% glucose, and 0.98% sucrose (http://www.suika-net.co.jp/nutri.html), while the content rate of sugar in the flesh of wild watermelon native to Botswana is 0.88% fructose, 0.45% glucose, and 0.05% or less of sucrose. As the wild watermelon native to Botswana have a low sugar concentration and are not sweet, they can be used across very wide applications. In particular, with respect to foods and drinks, it is possible to add to any food or drink.

In this specification, 'the wild watermelon extract' means that which is extracted from wild watermelon, including that which is ground up as wild watermelon fruits, juice of wild watermelon fruit, filtrate which filters that which is ground up as wild watermelon fruits or supernatant of the centrifugation of it. Moreover, it contains the extract which is extracted using a polar or nonpolar solvent, etc. The extraction solvent and the extraction method are not particularly limited, and any solvent or any method may be used as long as it is an extract of components derived from wild watermelon. The part for extraction is not particularly limited, but it is preferable to use flesh, especially preferable to use the flesh part whose hull, endothelium and seeds are removed.

An obtained extract may be used without modification, or in concentrated or dried form. And, a concentrated or dried one may be used to be dissolved or suspended in the appropriate solvent. It may be used by obtaining a fraction having active oxygen scavenging activity using liquid chromatography, etc., from the extract.

As cosmetics, skin lotions, various barrier creams, foundations and lipsticks, etc., are mentioned. Moreover, it includes bathwater agents, hair care products, for example, shampoos, hair conditioners, hair growth tonics and the like.

The active oxygen scavenging agent of the present disclosure contains wild watermelon extract. The blending quantity of the wild watermelon extract contained in the active oxygen scavenging agent can be changed according to its use and its object as well other active oxygen scavenging agents and is not particularly limited. And, the wild watermelon extract itself can be used as an active oxygen scavenging agent directly.

The active oxygen scavenging agent of the present disclosure can be used for improving or protecting the various disorders caused by active oxygen in vivo, adding an effective dose for active oxygen scavenging action to skin drugs for external use such as cosmetics.

Moreover, the effective dose for the active oxygen scavenging function means the dose which exerts an active oxygen scavenging function, or which can lower or eliminate active oxygen in vivo. The effective dose for active oxygen scavenging function varies according to objects, application parts, etc., and is not particularly limited as long as it exerts the above effect, but more specifically, can exemplify the range of ordinarily 0.025∼1.00% (hereinafter referred to as % by weight), more preferably, 0.05∼99%, especially preferably 1%∼99% with an example as a proportion of the containing amount of the botanical extract (equivalent in dry substances) of various compositions such as cosmetics, etc.

The present invention provides a moisturizing agent containing the wild watermelon extract. The wild watermelon extract, as shown in Example 2, has rich moisture comparable to glycerin. Therefore, it can also be used as a moisturizing agent. The moisturizing agent of the present invention (hereinafter called the agent) is not particularly limited, and can be used as a moisturizing agent for skin (including the face, limbs (pertaining to elbows, knees and heels), body, scalp) hair and nails, etc., for example. The moisturizing agent of the present disclosure may substantially consist of only wild watermelon extract or may be mixed with other components. As other components, if it can be dispensed as external medicine for skin (pertaining to drug medicines, cosmetics, fragrances, cleaning agents, bathwater agent, hair care products and other quasi drugs), various components can be used according to application site, use and intended purpose.

For example, other moisturizing agents, external bases, fragrances, thickeners, preservative agents, oil content, fatty acids, sterols, surface acting agents, water-soluble multiple alcohols, powders, silicones and other components, etc. Adding appropriately these components according to use and intended purpose, it can easily make the applicable form such as liquid form, solid form, paste form, gel form and emulsifying form etc.

Moreover, if it is a material of cosmetics, raw materials can be dispensed such as fats and oils, waxes, carbon hydrides, fatty acids, alcohols, esters, surface acting agents, whitening agents and moisturizing agents, etc., which are used ordinarily for cosmetics, etc., within the range without losing the moisturizing action. Concentration of the wild watermelon extract in the agent can be determined according to use and purpose of the agent.

In addition, the agent may be used for applying to the application part such as skin, hair and nails, etc., and can be used for combining external medicines for skin such as drug medicines and toiletries, etc. Therefore, the present disclosure provides a composition containing the above moisturizing agent of the present invention (the agent). The agent preferably contains wild watermelon extract as an active component. While the composition containing the agent is not particularly limited, it can be illustrated by examples, (1) cosmetics for skin containing creams, milky lotions, water lotions, pack agents, foundation (foundation creams), eye masks, ice creams, lipsticks and chapsticks, etc., (2) cosmetics for hair containing hair gels (for hair), hair sprays, hair liquids, hair tonics, hair mousse, pomade, stick pomade, rinses and hair treatments etc., (3) washing agents containing hair shampoos, body-wash, hand soaps and soaps (including both liquids and solids), etc., (4) bathwater agents (5) cosmetics for nails including nutrient creams for nails, manicures, (containing nail coats) and polish removers, etc., (6) hair growth tonic, etc. The composition containing moisturizing agents of the present disclosure can prevent the surface of the skin, etc., from drying out and exert a moisturizing effect by keeping or adhering the wild watermelon extract to the surface such as skin, etc., as a moisturizing agent.

The amount of a moisturizing agent mixed in a composition containing a moisturizing agent of the present disclosure can be determined appropriately according to the kind of composition, use and purpose, etc., and is not particularly limited. For example, the concentration (concentration is % by weight) is 0.1%∼100%, preferably 1%∼100%, more preferably 1%∼70%, more preferably 1%∼50%, especially preferably 3%∼30%. In addition, while the moisturizing agent of the present invention contains the extract of wild watermelon, the possibility of causing environmental pollution is not likely as such an extract is naturally-derived, even if it flows out to the natural world.

The extract of wild watermelon shown in the example, which contains citrulline 0.288mM, had about ten times higher active oxygen scavenging activity than an aqueous solution containing only citrulline of the same concentration. That is, the activity of citrulline which is occupied in the active oxygen scavenging activity of wild watermelon extract is about one tenth, and was shown that the contribution of components other than citrulline was far more significant. By this discovery, an extract of wild watermelon, a composition having antioxidation activity more than twice as high as the corresponding concentration of citrulline can be provided. As a composition, equivalent, cosmetics, bathwater agents and hair care products, etc., than a composition containing said active oxygen scavenging agent can be used.

Moreover, according to the present invention, the method to produce the active oxygen scavenging agent or moisturizing agent which is characterized by using the extract of wild watermelon as a part of the raw materials is provided. This can produce a novel active oxygen scavenging agent or moisturizing agent by blending or reacting the wild watermelon extract with other components. In this production method, it is similar to the production method of the active oxygen scavenging agent or moisturizing agent as is well known to those skilled in the art besides adding the extract of the wild watermelon as a raw material.

The present invention is explained as follows using examples, but it is not in any way limited to these examples.

### _Example 1

### (Activity of active oxygen scavenging)

Exodermises, endodermis, and seeds of wild watermelon native to Botswana were removed and the part of the flesh is obtained. After that, the flesh extract was obtained by crushing this flesh with a blender and by filtering it through gauze and Miracloth.

The active oxygen scavenging ability of the wild watermelon fruit extract is assayed by competitive reaction analysis using salicylic acid oxidation as an index. Specifically, 2mM salicylic acid and the wild watermelon fruit extract of various concentrations are dissolved in 50mM phosphate buffer (pH 7.4). After that, by adding 0.6mM of hydrogen peroxide, 0.15mM of FeEDTA and 0.26mM of ascorbic acid, hydroxyl radical is generated, and reacted at 25 degrees for 90 minutes. Quantitation of dihydroxyl benzoic acid (DHBA) generated by salicylic acid oxidation followed the method as set forth in the literature (Smirnofff and Cumbes (1989) Phytochemistry, 28: 1057-1060.)

As a result, by containing 25% of the fruit extract, oxidation of salicylic acid is inhibited by as much as 92% compared to the control added with only water; this has revealed that the wild watermelon fruit extract has exceptional active oxygen degradation ability. On the other hand, while 25% of the wild watermelon extract contains about 50mg/l (0.29mM) of citrulline, 50mg/l of citrulline solution was assayed for the active oxygen scavenging activity and salicylic acid oxidation was inhibited by only 6%. This shows a substance other than citrulline in the wild watermelon extract bears high active oxygen scavenging activity of the extract.

### _Example 2

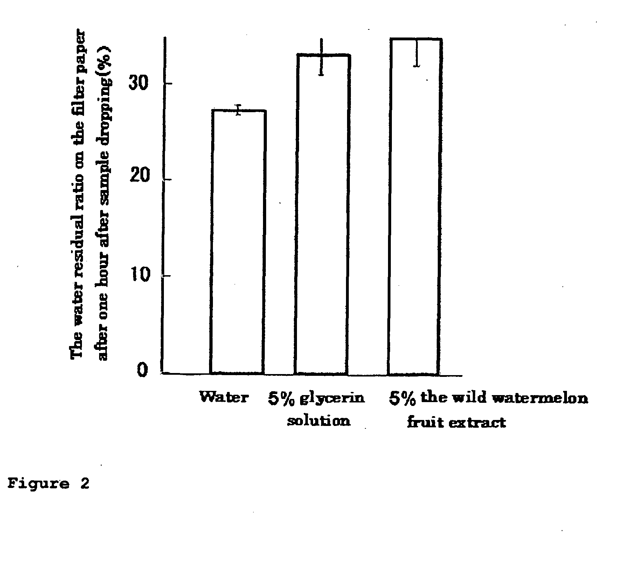

The moisturizing property of the wild watermelon fruit extract native to Botswana was estimated. Each 100µL of 5% aqueous solution of the wild watermelon extract and a comparative solution, 5% glycerol aqueous solution were dropped on the filter paper (Whatman filter paper 1, 42.5mm, Whatman Ltd.), and was left standing at a temperature of 25°C and at a relative humidity of 35%, and the water residual ratio was measured after two hours by weighing method. The result is shown in Figure 2.

In the case of dropping only water, 27% of the water was left but the water residual ratio could be raised to 33% by using glycerin which is widely used in cosmetics as a moisturizer. Compared with this, using the wild watermelon extract, the water residual ratio is 35%, water loss from the filter paper was confirmed to be suppressed more than glycerin and it is elucidated that wild watermelon fruit extract has a high moisturizing effect.

### _Example 3

The present invention can also be used as a bathwater agent. In one of extracts of wild watermelon of the present invention, the fruit juice of the flesh part of watermelon native to Botswana was dried, and was powdered and was made as a bathwater agent. Dissolving 10g of powder in about 200L of hot water, ten trial subjects with dry skin were asked to use it for one month. As a control, another ten people were asked to use a commercial bathwater agent for one month in a similar way. As a result, seven of ten who used the bathwater agent of the present invention answered that the dry feeling of skin had improved. On the other hand, four of ten control subjects answered that the dry feeling of skin had improved. Therefore, the bathwater agent of the present invention is considered to have an effect to improve the dry feeling of skin.

### _Example 4

After filtering one of the extracts of wild watermelon of the present invention, the fruit juice of the flesh part of wild watermelon native to Botswana through gauze, diluting it with a pure water to become 10% fruit juice and 10% ethanol, a lotion was prepared by filtering it again. Ten trial subjects with dry skin used it for one month. Another ten people were asked to use a commercial lotion for one month in a similar way as a control. As a result, nine of ten who used the lotion of the present invention answered that the dry feeling of skin had improved. On the other hand, four of ten trial subjects as controls answered that the dry feeling of skin had improved. Therefore, the lotion of the present invention is considered to have an effect to improve the dry feeling of skin.

### _Example 5

Components of wild watermelon native to Botswana were analyzed by a high-performance liquid chromatography. An analysis was performed as follows. An analysis of fructose, glucose and sucrose were performed as follows. About 5 volumes of 50% ethanol (V/V) was added to 4∼5g of crushed watermelon flesh, and sonication treatment was performed. A treatment condition was to use an ultrasonic generator (manufactured by Kokusai Electric Alpha Co., Ltd.) and to treat for half an hour. The volume of the sonicated sample was measured; it was filtered through a filter paper (Toyo Roshi, No. 5B). The filtrate was concentrated and dried by a rotatory evaporator and was dissolved in the appropriate volume of pure water. Filtering this solution through a membrane-filter (manufactured by Millipore Corporation, pore diameter 0.45µL), it was analyzed by a high-performance liquid chromatography.

Analysis condition is as follows.
- Model: LC-10ADvp (manufactured by Shimadzu Corporation)
- Detector: Differential refractometer RID-10A (manufactured by Shimadzu Corporation)
- Column: Wakosil 5NH2 φ_6mmX250mm (manufactured by Wako Pure Chemical Industries Ltd.)
- Solvent: Acetonitrile 75%/pure water 25%
- Flow rate: 1mL/minute

As a result of analysis, the content of each sugar per 100g of wild watermelon flesh native to Botswana was 0.88g of fructose, 0.45g of glucose and under a detectable level (0.05g) of sucrose.

### Industrial Applicability

The active oxygen scavenging agent and moisturizing agent of the present invention is available in industries of cosmetics, bathwater agents and hair care products, etc.

### Brief Description of the Drawings

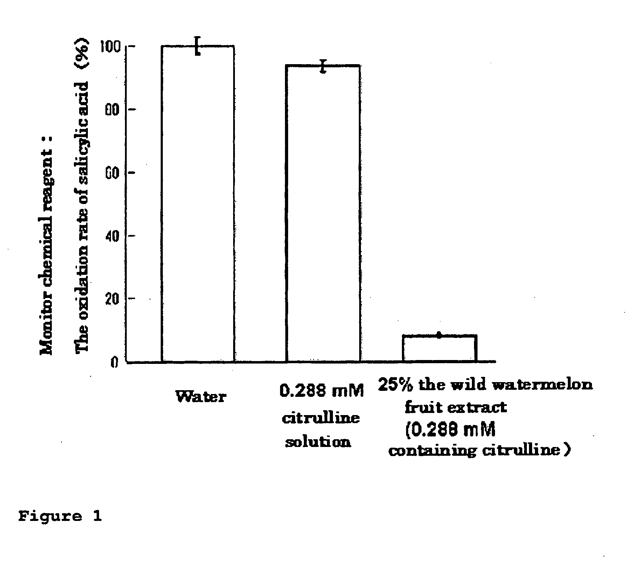

Figure 1 shows an antioxidation effect (hydroxyl radical suppressing activity) of the wild watermelon extract native to Botswana. Figure 1 shows, comparing active oxygen scavenging activity of extracts of wild watermelon with a single citrulline solution of its corresponding concentration, active oxygen scavenging activity of wild watermelon extract is about ten times higher than the single citrulline solution.
Figure 2 shows the moisturizing effect of wild watermelon extract native to Botswana. Figure 2 shows an effect comparable to glycerin, comparing the moisturizing effect of wild watermelon with that of glycerin,

## Claims

1. Use, as moisturizing agent, of the *Citrullus lanatus* (wild watermelon) extract native to Botswana,
- the wild watermelon extract being obtained by using *Citrullus lanatus* flesh, with the exception of hull, endothelium and seeds,
wherein the total of each concentration of fructose, glucose and sucrose is 2% or less weight/weight percent and
wherein the *Citrullus lanatus* (wild watermelon) extract has an oxygen scavenging activity about ten times higher than an aqueous solution containing only citrulline of the same concentration and
wherein the *Citrullus lanatus* (wild watermelon) extract has a rich moisture comparable to glycerin.

2. Use of claim 1, wherein the content rate of sugar in the flesh of *Citrullus lanatus* (wild watermelon) is 0.88% fructose, 0.45% glucose, and 0.05% or less of sucrose.

3. Use as moisturizing agent, of the *Citrullus lanatus* (wild watermelon) extract of claim 1 or 2, for the preparation of a moisturizing cosmetic composition.

4. Use according to any of claims 1 to 3, **characterized in that** the moisturizing agent is used to prepare a shampoo, a conditioner, a bathwater or a hair growth tonic.

## Patentansprüche

1. Verwendung des aus Botswana stammenden Extrakts von *Citrullus lanatus* (wilde Wassermelone) als Feuchtigkeitsspender,
wobei man den Extrakt von wilden Wassermelonen dadurch erhält, dass man das Fleisch von *Citrullus lanatus* mit Ausnahme von Schale, Endothel und Samen verwendet,
wobei die Konzentration an Fructose, Glucose und Sucrose insgesamt jeweils max. 2 Gew.-% beträgt, und
wobei der Extrakt von *Citrullus lanatus* (wilde Wassermelone) eine sauerstoffaufnehmende Wirksamkeit besitzt, die etwa zehnmal höher ist als bei einer wässrigen Lösung, die nur Citrullin in derselben Konzentration enthält, und wobei der Extrakt von *Citrullus lanatus* (wilde Wassermelone) eine mit Glycerin vergleichbare hohe Feuchtigkeit besitzt.

2. Verwendung nach Anspruch 1, wobei der Zuckeranteil im Fleisch von *Citrullus lanatus* (wilde Wassermelone) 0,88 % Fructose, 0,45% Glucose und max. 0,05% Sucrose beträgt.

3. Verwendung des Extrakts von *Citrullus lanatus* (wilde Wassermelone) als Feuchtigkeitsspender nach Anspruch 1 oder 2 für die Herstellung einer feuchtigkeitsspendenden kosmetischen Zusammensetzung.

4. Verwendung nach einem der Ansprüche 1 bis 3, **dadurch gekennzeichnet, dass** der Feuchtigkeitsspender zur Herstellung eines Shampoos, eines Conditioners, eines Badezusatzes oder eines Haarwuchs-Tonikums verwendet wird.

## Revendications

1. Utilisation, comme agent hydratant, d'un extrait de *Citrullus lanatus* (pastèque sauvage) originaire du Botswana, étant entendu
- que l'extrait de pastèque sauvage a été obtenu à partir de chair de *Citrullus lanatus,* à l'exclusion de l'écorce, de l'endothélium et des graines,
- que le total des concentrations de fructose, de glucose et de saccharose est égal ou inférieur à 2 % en poids,
- que l'extrait de *Citrullus lanatus* (pastèque sauvage) est doté d'une activité de piégeage de l'oxygène à peu près dix fois plus intense que celle d'une solution aqueuse ne contenant que de la citrulline en la même concenration,
- et que l'extrait de *Citrullus lanatus* (pastèque sauvage) offre un fort pouvoir hydratant, comparable à celui de la glycérine.

2. Utilisation conforme à la revendication 1, pour laquelle les teneurs en sucres de la chair de *Citrullus lanatus* (pastèque sauvage) sont les suivantes : 0,88 % de fructose, 0,45 % de glucose, et 0,05 % ou moins de saccharose.

3. Utilisation, comme agent hydratant, d'un extrait de *Citrullus lanatus* (pastèque sauvage) conforme à la revendication 1 ou 2, pour la préparation d'une composition cosmétique hydratante.

4. Utilisation conforme à l'une des revendications 1 à 3, **caractérisée en ce que** l'agent hydratant est utilisé pour préparer un shampooing, un conditionneur, des sels de bain ou un tonique capillaire.
